(19) Europäisches Patentamt
European Patent Office
Office européen des brevets

(11) **EP 3 276 356 B1**

(12) **EUROPEAN PATENT SPECIFICATION**

(45) Date of publication and mention
of the grant of the patent:
**27.11.2019 Bulletin 2019/48**

(51) Int Cl.:
***G01N 33/86*** (2006.01)

(21) Application number: **17183755.2**

(22) Date of filing: **28.07.2017**

(54) **METHOD FOR DETERMINING SEVERITY OF HEMOPHILIA AND BLOOD SPECIMEN ANALYZER**

VERFAHREN ZUR BESTIMMUNG DER SCHWERE VON HÄMOPHILIE UND BLUTPROBENANALYSATOR

PROCÉDÉ PERMETTANT DE DÉTERMINER LA SÉVÉRITÉ DE L'HÉMOPHILIE ET ANALYSEUR D'ÉCHANTILLON DE SANG

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.07.2016 JP 2016148459**

(43) Date of publication of application:
**31.01.2018 Bulletin 2018/05**

(73) Proprietors:
• **SYSMEX CORPORATION**
**Kobe-shi**
**Hyogo 651-0073 (JP)**
• **Public University Corporation**
**Nara Medical University**
**Nara 634-8521 (JP)**

(72) Inventors:
• **Shima, Midori**
**Nara, 634-8521 (JP)**
• **Nogami, Keiji**
**Nara, 634-8521 (JP)**
• **Matsumoto, Tomoko**
**Nara, 634-8521 (JP)**
• **Tabuchi, Yuka**
**Hyogo, 651-0073 (JP)**
• **Arai, Nobuo**
**Hyogo, 651-0073 (JP)**

(74) Representative: **Hoffmann Eitle**
**Patent- und Rechtsanwälte PartmbB**
**Arabellastraße 30**
**81925 München (DE)**

(56) References cited:
**WO-A1-2011/159820      US-A1- 2013 344 519**
**US-A1- 2016 178 651**

• **A K ET AL: "The measurement of low levels of factor VIII or factor IX in hemophilia A and hemophilia B plasma by clot waveform analysis and thrombin generation assay", JOURNAL OF THROMBOSIS AND HAEMOSTASIS, vol. 4, no. 2, 1 February 2006 (2006-02-01), pages 377-384, XP055143046,**
• **MEERA CHITLUR: "Challenges in the laboratory analyses of bleeding disorders", THROMBOSIS RESEARCH, TARRYTOWN, NY, US, vol. 130, no. 1, 13 March 2012 (2012-03-13), pages 1-6, XP028518184, ISSN: 0049-3848, DOI: 10.1016/J.THROMRES.2012.03.011 [retrieved on 2012-03-17]**
• **K. YADA ET AL: "The mild phenotype in severe hemophilia A with Arg1781His mutation is associated with enhanced binding affinity of factor VIII for factor X :", THROMBOSIS AND HAEMOSTASIS, vol. 109, no. 6, 7 March 2013 (2013-03-07), pages 1007-1015, XP055409914, DE ISSN: 0340-6245, DOI: 10.1160/TH12-10-0762**

**Description**

TECHNICAL FIELD

The present invention relates to a method for determining severity of hemophilia and a blood specimen analyzer.

BACKGROUND

Hemophilia is a hemorrhagic disease caused by congenital defect or dysfunction of coagulation factor VIII (FVIII) or coagulation factor IX (FIX). Hemophilia is called hemophilia A when it is caused by FVIII. Hemophilia is called hemophilia B when it is caused by FIX. In hemophilic patients, bleeding symptoms are found in deep tissues such as intraarticular tissues or intramuscular tissues, and intracranial hemorrhage also occurs in severe cases.

The severity of hemophilia is classified based on FVIII activity or FIX activity in the blood. Specifically, patients with an activity of less than 1% are classified as severe, patients with an activity of 1% or more and less than 5% are classified as moderate, and patients with an activity of 5% or more and less than 40% are classified as mild, based on FVIII activity or FIX activity of healthy subjects as 100%. Patients with severe hemophilia exhibit bleeding symptoms at a frequency significantly higher than patients with moderate and mild cases. However, in recent years, it has been understood that, among patients with severe hemophilia, there is a difference in clinical severity and in occurrence probability of inhibitors to be described later, between patients with a particularly low FVIII activity (less than 0.2%; Very-Severe Haemophilia A; VS-HA) and other patients (0.2% or more and less than 1%; Modestly-Severe Haemophilia A; MS-HA).

In order to prevent bleeding symptoms in patients with severe hemophilia, a replacement therapy that gives administration of a coagulation factor preparation to patients is useful. However, antibodies (inhibitors) against such coagulation factors may occur in 10 to 15% of patients with hemophilia A and 1 to 3% in patients with hemophilia B. In patients with an inhibitor value of less than 5 BU (Bethesda unit), the inhibitor may transiently disappear. On the other hand, in patients with an inhibitor value of 5 BU or more, inhibitors tend to remain in the blood. For such patients, it is difficult to manage hemostasis with a coagulation factor preparation. Thus, it is forced to change the treatment policy by switching to immune tolerance therapy, administration of bypass preparation, or the like. Patients with inhibitors can be more severe than VS-HA patients. Therefore, from the viewpoint of selecting an appropriate treatment policy, it is important to detect the appearance of the inhibitor with high accuracy at an early stage.

A method for examining the presence or absence of the inhibitor by a blood test is known. For example, Matsumoto et al., A putative inhibitory mechanism in the tenase complex responsible for loss of coagulation function in acquired hemophilia A patients with anti-C2 autoantibodies, Thorombosis and Haemostasis 107.2/2012 describes that a plasma specimen of a moderate hemophilia A patient (Moderate type HA, 2.1 $\pm$ 0.9 IU/dl) and a plasma specimen of a hemophilia A patient in which the antibody has appeared (Type 2) are distinguished using the minimum value mini ($|min1|$ : maximum absolute value) of a first derivative curve of a coagulation curve.

Furthermore, Matsumoto et al. (J Thromb Haemost 4(2): 377-84; 2006) describe the measurement of low levels of factor VIII or factor IX in hemophilia A and hemophilia B plasma by clot waveform analysis and thrombin generation assay. US 2013/344519 A1 relates to methods and systems for assessment of turbidity kinetics (waveform analysis) in coagulation testing. WO2011159820 A1 describes a method for measuring a coagulation event by the continuous recording of the coagulation process. Chitlur (Thromb Res 130(1): 1-6; 2012) describes the thrombin generation test, thromboelastography, the activated partial thromboplastin time waveform analysis and the CloFAL which measure the production of thrombin, as well as the kinetics of clot formation. Yada et al. (Thromb Haemost 109(6): 1007-15; 2013) performed, *inter alia,* clot waveform analyses to suggest that the mild phenotype in severe hemophilia A with Arg1781His mutation is associated with enhanced binding affinity of factor VIII for factor X. US 2016/178651 A1 relates to a blood sample determination method and blood sample analyzer wherein at least one parameter regarding the derivative of a clot waveform is used to determine whether a blood sample is suspected to be a sample derived from a subject having lupus anticoagulant or a coagulation factor inhibitor.

SUMMARY OF THE INVENTION

An object of the present invention is to provide a method for determining severity of hemophilia that can determine with high accuracy a blood specimen of a severe hemophilia patient such as a blood specimen of a VS-HA patient and a blood specimen of a hemophilia patient, who is more severe than a VS-HA patient, in which the antibody against a coagulation factor has appeared.

The present invention provides a method for determining severity of hemophilia, the method including the steps of: coagulating a blood specimen to acquire a coagulation waveform; acquiring an average change rate of a coagulation rate from the coagulation waveform, wherein the everge change rate of the coagulation rate is calculated based on a waverform of rate obtained by differntiating the coagulation waveform; and determining the severity of hemophilia in the blood specimen based on the average change rate of the coagulation rate.

The present invention also provides a blood specimen analyzer including a measurement sample preparing unit configured to prepare a measurement sample including a blood specimen and a coagulation time measuring reagent, an information acquisition unit configured to acquire a coagulation waveform from the prepared measurement sample, and a control unit, wherein the control unit is configured to control the measurement sample preparing unit so as to prepare the measurement sample from the blood specimen and the coagulation time measuring reagent, is configured to acquire an average change rate of a coagulation rate based on the coagulation waveform, wherein the average change rate of the coagulation rate is calculated based on a waveform of rate obtained by differntiating the coagulation waveform and is configured to output information on severity of hemophilia based on the average change rate of the coagulation rate.

It is possible to provide a method for determining severity of hemophilia that can determine with high accuracy a blood specimen of a severe hemophilia patient, especially a VS-HA patient, and a blood specimen of a hemophilia patient, who is more severe than a VS-HA patient, in which the antibody against a coagulation factor has appeared.

## BRIEF DESCRIPTION OF THE DRAWINGS

Fig. 1A is an example of a coagulation waveform obtained by measuring transmittance of a normal plasma specimen.
Fig. 1B is an example of a waveform of rate obtained by primarily differentiating the coagulation waveform in Fig. 1A.
Fig. 2 is an example of a waveform of coagulation acceleration obtained by secondarily differentiating a coagulation waveform.
Fig. 3 is an example of a possible approximation by a method of the present embodiment.
Fig. 4 is a perspective view showing the configuration of an appearance of a blood specimen analyzer.
Fig. 5 is a plan view of an inside of a measurement unit of the blood specimen analyzer when viewed from above.
Fig. 6 is a diagram showing the configuration of the measurement unit of the blood specimen analyzer.
Fig. 7 is a diagram showing the configuration of a lamp unit provided in the measurement device.
Fig. 8A is a diagram showing the configuration of a detection unit provided in the measurement device.
Fig. 8B is a diagram showing the configuration of the detection unit provided in the measurement device.
Fig. 8C is a diagram showing the configuration of the detection unit provided in the measurement device.
Fig. 8D is a diagram showing the configuration of the detection unit provided in the measurement device.
Fig. 9 is a diagram showing the functional configuration of a control device of the blood specimen analyzer.
Fig. 10 is a diagram showing the hardware configuration of a control device of the blood specimen analyzer.
Fig. 11 is a flowchart showing measurement processing of a blood specimen by the blood specimen analyzer.
Fig. 12 is a flowchart showing analysis processing of a blood specimen by the blood specimen analyzer.
Fig. 13 is a view showing an example of a screen for displaying an analysis result by the blood specimen analyzer.
Fig. 14 is a diagram showing a discrimination result using parameters of the present embodiment.
Fig. 15 is a diagram showing a discrimination result using conventional parameters.

## DETAILED DESCRIPTION OF THE PREFERRED EMBODIMENTS

A method of determining the severity of hemophilia includes a step of coagulating a blood specimen to acquire a coagulation waveform.

Examples of the blood specimen include whole blood and plasma, and the blood specimen is preferably plasma. The blood specimen is preferably a blood specimen derived from a patient suspected of being a VS-HA patient. A known anticoagulant commonly used for coagulation test may be added to the blood specimen. Examples of the anticoagulant include trisodium citrate. Prior to preparation of a measurement sample, the blood specimen may be heated to a temperature suitable for coagulation reaction (for example, 36°C or more and 38°C or less) in advance.

The method for coagulating the blood specimen is not particularly limited, and can be performed by a method known to those skilled in the art. For example, it can be performed by contacting a blood specimen with a coagulation time measuring reagent to prepare a measurement sample, and coagulating the blood specimen in the measurement sample.

The coagulation time measuring reagent should be any reagent for measuring coagulation time based on the measurement principle known in the art. Examples of the reagent include reagents for measuring at least one of activated partial thromboplastin time, prothrombin time, diluted prothrombin time, diluted activated partial thromboplastin time, kaolin clotting time, diluted Russell viper venom time, thrombin time, and diluted thrombin time. The coagulation time measuring reagent is preferably a reagent for measuring activated partial thromboplastin time. Commercially available coagulation time measuring reagents and reagent kits may be used.

It is preferable that the coagulation time measuring reagent contains a coagulation system activator. The coagulation system activator should be any substance that activates any coagulation factor involved in the coagulation system. Examples of the coagulation system activator include ellagic acid, silica, kaolin, celite, tissue factor, thrombin, viper venom, and the like. Ellagic acid may be in a state of forming a chelate with a metal ion. The tissue factor may be a tissue factor derived from rabbit brain or human placenta, or may be a recombinant tissue factor. Examples of the viper venom include Russell viper venom, Texturing viper venom, Ecarin viper venom, and the like. These coagulation system activators may be used alone or in combination of two or more kinds thereof. Usually, commercially available coagulation time measuring reagents and reagent kits contain any coagulation system activator, depending on the kind of coagulation time to be measured.

The final concentration of the coagulation system activator in the measurement sample can be appropriately determined according to the kind of the coagulation system activator. When the coagulation system activator is ellagic acid, the final concentration of ellagic acid in the measurement sample is usually 3.5 $\mu$M or more and 150 $\mu$M or less, and preferably 10 $\mu$M or more and 50 $\mu$M or less. When the coagulation system activator is a tissue factor, the final concentration of the tissue factor in the measurement sample is usually 0.4 $\mu$g/mL or more and 0.7 $\mu$g/mL or less, and preferably 0.5 $\mu$g/mL or more and 0.6 $\mu$g/mL or less.

Since a phospholipid promotes coagulation reaction, the coagulation time measuring reagent may further contain a phospholipid. Examples of the phospholipid include phosphatidylethanolamine (PE), phosphatidylcholine (PC) and phosphatidylserine (PS). In the present aspect falling within the scope of the invention one, preferably two, more preferably all kinds of phospholipids selected from PE, PC and PS can be added to the coagulation time measuring reagent. The phospholipid may be a naturally occurring phospholipid or a synthetic phospholipid. Among them, synthetic phospholipids or naturally occurring phospholipids purified to have a purity of 99% or more are preferred. The fatty acid side chains of PE, PC and PS are not particularly limited, and examples thereof include palmitic acid, oleic acid, stearic acid, and the like. Among them, oleic acid is preferable. In the present aspect falling within the scope of the invention, the phospholipid is preferably in the form of a liquid in which the phospholipid is dissolved in a suitable solvent.

The final concentration of the phospholipid in the measurement sample can be appropriately determined depending on the kind of the phospholipid. When the phospholipid is PE, the final concentration of the phospholipid in the measurement sample is usually 1 $\mu$g/mL or more and 150 $\mu$g/mL or less, and preferably 5 $\mu$g/mL or more and 50 $\mu$g/mL or less. When the phospholipid is PC, the final concentration of the phospholipid in the measurement sample is usually 1 $\mu$g/mL or more and 100 $\mu$g/mL or less, and preferably 5 $\mu$g/mL or more and 80 $\mu$g/mL or less. When the phospholipid is PS, the final concentration of the phospholipid in the measurement sample is usually 0.1 $\mu$g/mL or more and 50 $\mu$g/mL or less, and preferably 1 $\mu$g/mL or more and 10 $\mu$g/mL or less. When using two or more kinds of the phospholipids, the total concentration of each of the phospholipid in the measurement sample is usually 5 $\mu$g/mL or more and 400 $\mu$g/mL or less, and preferably 20 $\mu$g/mL or more and 100 $\mu$g/mL or less.

The coagulation time measuring reagent preferably contains a phospholipid and a coagulation system activator. Examples of the coagulation time measuring reagent containing a phospholipid and a coagulation system activator include an activated partial thromboplastin time (APTT) measuring reagent. In this case, the coagulation system activator is preferably a substance that activates a contact factor of the intrinsic coagulation pathway, such as ellagic acid, silica, kaolin and celite.

Calcium ions are required to initiate blood coagulation in the measurement sample. In the present aspect falling within the scope of the invention, calcium ions are provided to the measurement sample by using an aqueous solution containing calcium ions for preparation of the measurement sample. As the aqueous solution containing calcium ions, an aqueous solution of a calcium salt is preferable, and examples thereof include an aqueous calcium chloride solution, an aqueous calcium lactate solution, and the like. The calcium ion content in the measurement sample may be an amount sufficient to cause coagulation, and for example, it is usually 2 mM or more and 20 mM or less, and preferably 4 mM or more and 10 mM or less, in terms of calcium chloride concentration. Hereinafter, the aqueous solution containing calcium ions is also called a "calcium solution".

Since coagulation starts when a calcium solution is added, it is preferable to add the calcium solution last in the preparation of the measurement sample. The preparation procedure of the measurement sample is as follows. The measurement sample can be prepared by, firstly, mixing a blood specimen with a coagulation time measuring reagent and then mixing the resulting mixture with a calcium solution. Alternatively, firstly, a measurement sample may be prepared by mixing a blood specimen with a coagulation time measuring reagent and then mixing the resulting mixture with a calcium solution. When using a commercially available prothrombin time (PT) measuring reagent, the reagent contains a tissue factor and calcium ions, and thus a measurement sample can be prepared by mixing a blood specimen with a PT measuring reagent. By which procedure to prepare the measurement sample may be determined according to the coagulation time measuring reagent to be used.

In this aspect falling within the scope of the invention, the mixture may be incubated under conditions suitable for coagulation reaction before adding the calcium solution. Examples of the suitable conditions include conditions for incubating at a temperature of 35°C or more and 40°C or less for a time period of 2 minutes or more and 5 minutes or less. The preparation of the measurement sample may be carried out by a manual method or may be carried out by a fully automatic measurement device. Examples of the device include the CS series of a fully automated blood coagulation measurement device (Sysmex Corporation), and the like.

When using the coagulation time measuring reagent containing a phospholipid and a coagulation system activator, it is preferable to prepare a measurement sample as follows. First, a blood specimen is mixed with the coagulation time measuring reagent containing a phospholipid and a coagulation system activator. Next, the resulting mixture is mixed with a calcium solution.

In the method of the present aspect falling within the scope of the invention, a coagulation waveform is acquired using the measurement sample prepared as described above. A coagulation waveform is a waveform representing a change with time in the optical characteristics and the like of a blood specimen caused accompanying the progress of coagulation of the blood specimen. In the present aspect falling within the scope of the invention, the coagulation waveform may be acquired by an optical measurement method. An example of the optical measurement method includes a method of acquiring optical information such as transmittance by irradiating the measurement sample with light. The measurement may be performed by a fully automatic measurement device. For example, the CS series of a fully automated blood coagulation measurement device (Sysmex Corporation) can measure optical information such as transmittance.

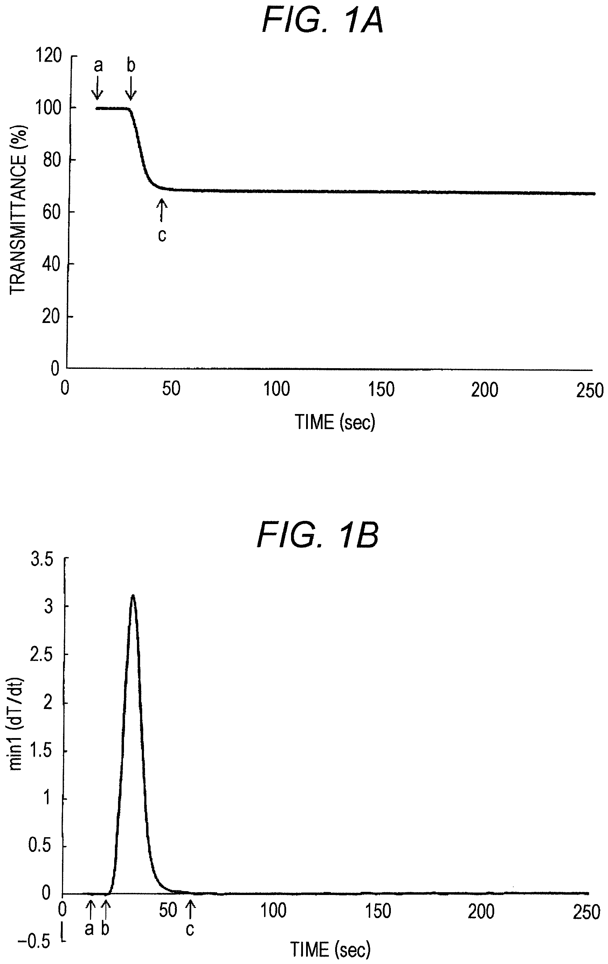

The acquisition of the optical information is carried out continuously or intermittently from the start point to the completion of coagulation. Based on the optical information measured continuously or intermittently in the process of coagulation, it is possible to acquire parameters related to the differentiation of coagulation waveform to be described later at any time point or time in the process. The start point refers to a time point at which acquisition of data of each plot constituting the coagulation waveform is started, in order to acquire the average change rate of a coagulation rate to be described later. The start point is not particularly limited as long as it does not hinder the acquisition of the average change rate of the coagulation rate; however, it is preferable to set the start point before the time point when the coagulation rate becomes the maximum. For example, the start point can be set to the measurement starting point of coagulation time (point a in Figs. 1A and 1B) or the starting point of coagulation (point b in Figs. 1A and 1B), or a time point indicated by the numerical value obtained by adding or subtracting an arbitrary coefficient to or from the numerical value indicating the time point. The start point is preferably set to the measurement starting point of coagulation time.

The measurement time may be determined from the range of usually 5 seconds or more and 1800 seconds or less, and preferably 10 seconds or more and 600 seconds or less. In the method of the present aspect falling within the scope of the invention, when normal plasma (plasma obtained from a healthy subject) is used as a blood specimen, coagulation is usually completed within 30 seconds from the time of preparation of the measurement sample.

The coagulation waveform is preferably acquired from optical information obtained by irradiating the measurement sample with light. Examples of the optical information include the amount of scattered light, transmittance and absorbance measured continuously or intermittently, and the like. In this case, the coagulation waveform is a waveform representing a change with time in the amount of scattered light, transmittance or absorbance. The light to be irradiated to the measurement sample may be light which is usually used for measuring coagulation time, and is for example, light having a wavelength of around 660 nm, and preferably, light having a wavelength of 660 nm. A light source is not particularly limited, and examples thereof include a light emitting diode, a halogen lamp, and the like. The coagulation waveform acquired in the present aspect falling within the scope of the invention includes the coagulation waveform curve itself and the data of each plot constituting the coagulation waveform. Examples of the data of each plot constituting the coagulation waveform include the time from the start point and the measurement value of the optical specification of the measurement sample at the time point.

An example of the coagulation waveform obtained by the method of the present aspect falling within the scope of the invention will be described with reference to Fig. 1A. In the coagulation waveform shown in Fig. 1A, a point a is the measurement starting point of coagulation time, a point b is the point of fibrin precipitation (starting point of coagulation), and a point c is the end point of coagulation. In a general coagulation time measuring method, the time to precipitate fibrin is defined as coagulation time. In Fig. 1A, the time between a and b represents coagulation time. Since coagulation proceeds by the action of the coagulation time measuring reagent, the transmittance of the measurement sample decreases as shown in a to c in Fig. 1A.

The method for determining the severity of hemophilia includes the step of acquiring an average change rate of the coagulation rate from the coagulation waveform.

The average change rate of the coagulation rate is calculated as described in the appended claims. It reflects the average change rate of the coagulation rate with respect to the time from the start point. For example, the average change rate of the coagulation rate may be an approximate value reflecting the average change rate of the coagulation rate. In the present embodiment, a curve obtained by differentiation of the coagulation waveform, parameters related to differentiation, and the like are acquired, and the average change rate of the coagulation rate is acquired using them.

The curve obtained by differentiation of the coagulation waveform is preferably a primary differential curve (waveform of rate) obtained by primarily differentiating the coagulation waveform. With reference to Fig. 1B, the waveform of rate will be described. When the coagulation waveform of Fig. 1A is primarily differentiated, a waveform showing the rate of coagulation shown in Fig. 1B is obtained. In Fig. 1B, the waveform is represented so that the coagulation rate (rate between a and c) is a positive value; however, it may be represented so that the coagulation rate is a negative value. That is, a waveform in which the plus or minus of the vertical axis is inverted from the waveform in Fig. 1B may be acquired. Points a to c in Fig. 1B correspond to the points a to c in Fig. 1A. In Fig. 1B, the point c that represents the end point of coagulation is a point at which the increased rate is zero.

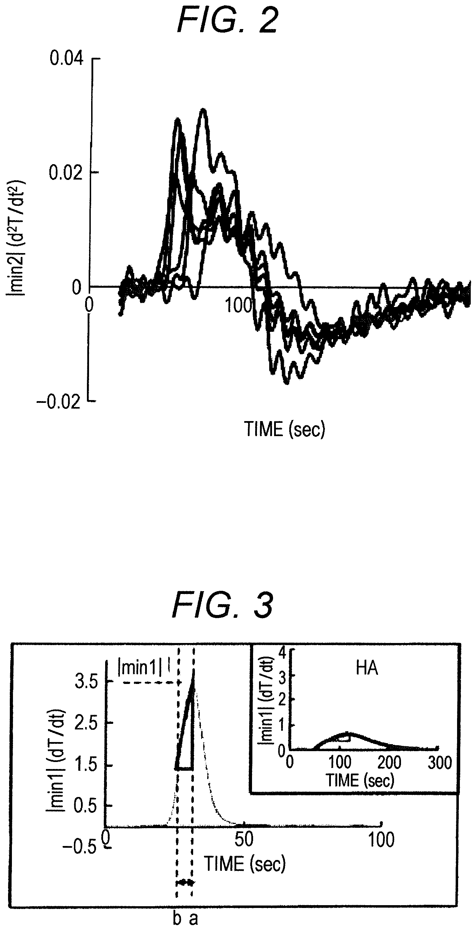

It is preferable that the curve obtained by differentiation of the coagulation waveform is not a secondary differential curve obtained by differentiating the coagulation waveform twice. This is because a second derivative curve easily includes noise, and shows a non-uniform waveform (for example, Fig. 2), so that accurate information may not be obtained in the microdetermination of coagulation factors. It is assumed that a curve obtained by differentiating the coagulation waveform three or more times easily includes noise as well. Therefore, it is particularly preferable that the curve obtained by differentiation of the coagulation waveform is a primary differential curve.

The parameters related to differentiation of the coagulation waveform are not particularly limited as long as they are values indicating at least one such as a coagulation rate that is obtained by differentiating the coagulation waveform at least once. Examples of the parameters related to differentiation of the coagulation waveform include a coagulation rate (the slope of the coagulation waveform), the maximum coagulation rate (the maximum value of the slope of the coagulation waveform) ($|min1|$), a time until the coagulation rate becomes the maximum (time to $|min1|$), a time until the coagulation rate becomes a times ($0 < a < 1$) the maximum (time to $a \times |min1|$), an average change rate of the coagulation rate, a time until coagulation acceleration (the slope of the waveform of the rate) becomes the maximum (time to $|min2|$), a time until coagulation acceleration becomes a times ($0 < a < 1$) the maximum (time to $a \times |min2|$) and their approximate values, and the like. The number of the parameters related to differentiation of the coagulation waveform may be one, or two or more. The parameters related to differentiation of the coagulation waveform may be a value obtained by combination of two or more parameters, and examples of the value include the sum, difference, product and ratio of at least two of the parameters exemplified above, and the like.

The average change rate of the coagulation rate is calculated based on the waveform of rate obtained by differentiating the coagulation waveform. An example of the average change rate of the coagulation rate includes a magnitude of the slope of a straight line connecting two points on the waveform of rate. The two points on the waveform of rate are selected from an arbitrary point on the waveform of rate and a peripheral point close to the arbitrary point. Both of these two points are preferably selected from the range of $(0, f(0))$ to (time to $|min1|$, $|min1|$) with representation of arbitrary points on the waveform of rate as (time, coagulation rate) = $(x, f(x))$. Time $x = 0$ is a start point. More preferably, the average change rate of the coagulation rate is the magnitude of the slope of a straight line connecting the point (time to $|min1|$, $|min1|$) and the point (time to $|min2|$, f(time to $|min2|$)). Each of time to $|min1|$, f(time to $|min1|$), time to $|min2|$, and f(time to $|min2|$) can be represented by their approximate values. Since f(time to $|min2|$) can be approximated as $1/2 \times |min1|$, $1/2 \times |min1|$ can be used as f(time to $|min2|$). The time (time to $1/2 \times |min1|$) until the coagulation rate becomes $1/2 \times |min1|$ can be approximated as time to $|min2|$. Therefore, as the point (time to $|min2|$, f(time to $|min2|$)), for example, the point (time to $|min2|$, $1/2 \times |min1|$), the point (time to $1/2 \times |min1|$, $1/2 \times |min1|$) or the like can be used.

With reference to Fig. 3, an example of a possible approximation by the method of the present embodiment will be described in detail. The waveform in the lower left diagram in Fig. 3 is a waveform of rate derived from a blood specimen of a healthy person, and the upper right diagram in Fig. 3 is the waveform of rate derived from a hemophilia A patient. In Fig. 3, the broken line of a represents a straight line parallel to the vertical axis passing through the point (time to $|min1|$ 0), and the intersection of the straight line and the waveform of rate corresponds to the point (time to ($min1|$, $|min1|$). On the other hand, the broken line of b represents a straight line parallel to the vertical axis passing through the point (time to $|min2|$, 0), and the intersection of the straight line and the waveform of rate corresponds to the point (time to $|min2|$, f(time to $|min2|$)). Since f(time to $|min2|$) can be approximated by $1/2 \times |min1|$, the average change rate of the coagulation rate is calculated as a magnitude of the slope of a straight line connecting two points, the point (time to $|min1|$, $|min1|$) and the point (time to $|min2|$, $1/2 \times |min1|$). The triangle shown in Fig. 3 is a conceptual diagram of the approximation.

Though not shown in Fig. 3, the above approximation can be similarly applied to patients with severe hemophilia, especially MS-HA patients and VS-HA patients as well as hemophilia patients (HA-inh) having the antibody.

In another viewpoint, the average change rate of the coagulation rate is preferably calculated using the maximum coagulation rate ($|min1|$) and the time until the coagulation rate becomes the maximum (time to $|min1|$), and is more preferably calculated further using the time until the coagulation acceleration becomes the maximum (time to $|min2|$).

In a particularly preferred embodiment, the average change rate of the coagulation rate is calculated by the following Expression 1.

[Mathematical Expression 1]

$$(1/2 \times |min1|)/(time\ to\ |min1| - time\ to\ |min2|)...Expression\ 1$$

Using Expression 1 above, it is possible to calculate the average change rate of the coagulation rate by using only those which can be easily acquired among the parameters related to differentiation. This is based on the fact empirically found by the present inventors that the time until the coagulation rate becomes $1/2 \times |min1|$ (time to $1/2 \times |min1|$) can be approximated by time to $|min2|$, when the average change rate of the coagulation rate is calculated by using a blood specimen of a severe hemophilia patient, a blood specimen of a hemophilia patient in which the antibody has appeared, and a blood specimen of a healthy person. From this viewpoint, according to the method of the present embodiment, it can also be said that the time until the coagulation acceleration becomes the maximum is the time before the coagulation rate becomes the maximum and until the coagulation rate becomes 1/2 of the maximum coagulation rate.

The method for determining the severity of hemophilia includes the step of determining the severity of hemophilia in the blood specimen based on the average change rate of the coagulation rate.

Hemophilia may be either hemophilia A or hemophilia B, and is preferably hemophilia A.

In the present embodiment, when the average change rate of the coagulation rate is equal to or less than the reference value, it is determined that there is a high possibility of the antibody against a coagulation factor having appeared in the blood specimen, and when the average change rate exceeds the reference value, it is determined that there is not a high possibility of the antibody against a coagulation factor having appeared in the blood specimen. The phrase "the antibody has appeared" means that the antibody is present in the blood at least at a detectable level, and may mean the state in which the blood antibody level is 5 BU or more. Such a determination result can be data that supports a doctor to diagnose that inhibitors have appeared in the blood of a hemophilia patient. When the antibody has appeared as above, it can be considered that hemophilia is more severe than VS-HA patients. In contrast, when the average change rate of the coagulation rate exceeds the reference value, no inhibitor has appeared in the blood of a hemophilia patient, but it can be data that supports a doctor to diagnose the patient as severe.

In severe hemophilia, especially VS-HA with a factor VIII activity of less than 0.2 IU/dl, and in hemophilia in which the antibody has appeared, the coagulation time is markedly prolonged in the APTT test in both hemophilia cases, and both hemophilia cases are below the quantitation limit in the quantitative examination of coagulation factor. Thus, it is difficult to simply detect inhibitors by such a general test. On the other hand, when inhibitors occur in hemophilia patients, it is difficult to manage hemostasis with factor VIII preparations commonly used in replacement therapy. Thus, it is necessary to consider immune tolerance therapy or administration of bypass preparation, and it is important to detect inhibitor expression in the choice of treatment at an early stage, with high specificity and high reliability. According to the method of the present embodiment, as described above, VS-HA with a factor VIII activity of less than 0.2 IU/dl can be discriminated from hemophilia in which the antibody has appeared. Thus, it is possible to select a treatment policy suitable for each patient. This is an unexpected result from the prior art. The case of hemophilia B is also the same only by replacing factor VIII with factor IX.

The reference value is not particularly limited, and can be appropriately set in advance by a person skilled in the art. For example, a coagulation waveform is acquired in advance using each of a blood specimen from a severe hemophilia A patient group and a blood specimen from a hemophilia patient group in which the antibody has appeared, and the value of Expression 1 above is obtained based on the obtained coagulation waveform. Then, it is possible to set a value that can most accurately classify the severe hemophilia patient group, particularly a VS-HA patient group, and the hemophilia patient group in which the antibody has appeared.

[2. Blood Specimen Analyzer]

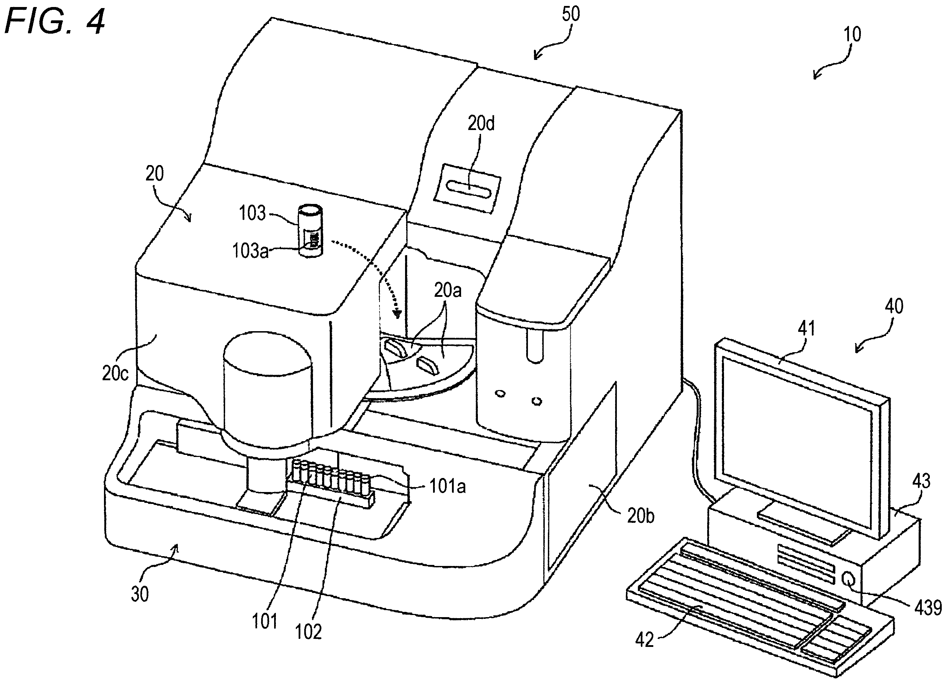

An example of a blood specimen analyzer will be described below with reference to the drawings. However, the present embodiment is not limited to this example. As shown in Fig. 4, a blood specimen analyzer 10 includes a measurement device 50 for preparing and measuring a measurement sample; a control device 40 for analyzing measurement data acquired by the measurement device 50 and providing an instruction to the measurement device 50. The measurement device 50 includes a measurement unit 20 for acquiring optical information from the measurement sample and a specimen transporting section 30 arranged in front of the measurement unit 20.

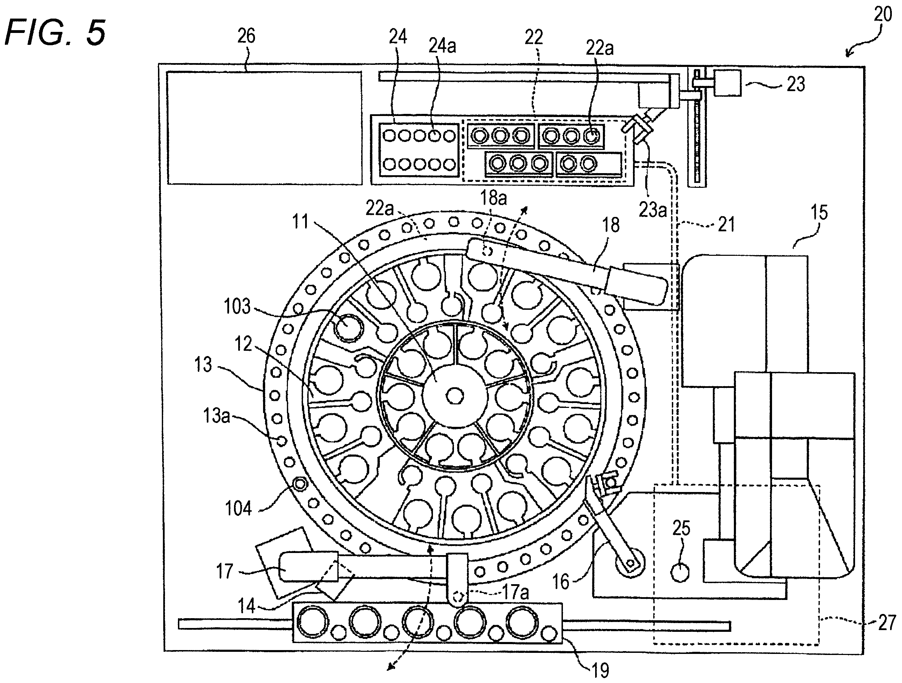

The measurement unit 20 is provided with lids 20a and 20b, a cover 20c, and a power button 20d. A user can open the lid 20a and replace a reagent container 103 placed in reagent tables 11 and 12 (see Fig. 5) with a new reagent container 103, or a user can newly add another reagent container 103. To the reagent container 103 is attached a barcode label 103a printed with a barcode including the kind of the reagent to be accommodated and a reagent ID made up of serial number provided to the reagent.

The user can open the lid 20b and replace a lamp unit 27 (see Fig. 5). The user can also open the cover 20c and replace a piercer 17a (see Fig. 5). The specimen transporting section 30 transports a specimen container 101 supported by a specimen rack 102 to an aspiration position by the piercer 17a. The specimen container 101 is hermetically sealed by a rubber lid 101a.

When using the blood specimen analyzer 10, the user first presses the power button 20d of the measurement unit 20 to activate the measurement unit 20, and the user presses a power button 439 of the control device 40 to activate the control device 40. When the control device 40 is activated, a log-on screen is displayed on a display unit 41. The user inputs the user name and the password on the log-on screen to log on to the control device 40, and the user starts using the blood specimen analyzer 10.

(Configuration of Measurement Device)

The configuration of the measurement device 50 will be described below. As shown in Fig. 5, the measurement unit 20 includes reagent tables 11 and 12, a cuvette table 13, a barcode reader 14, a cuvette supply section 15, a catcher 16, a specimen dispensing arm 17, a reagent dispensing arm 18, an urgent specimen setting section 19, an optical fiber 21, a detecting section 22, a cuvette transfer section 23, a warming section 24, a disposal port 25, a fluid section 26, and a lamp unit 27.

(Measurement Sample Preparing Unit)

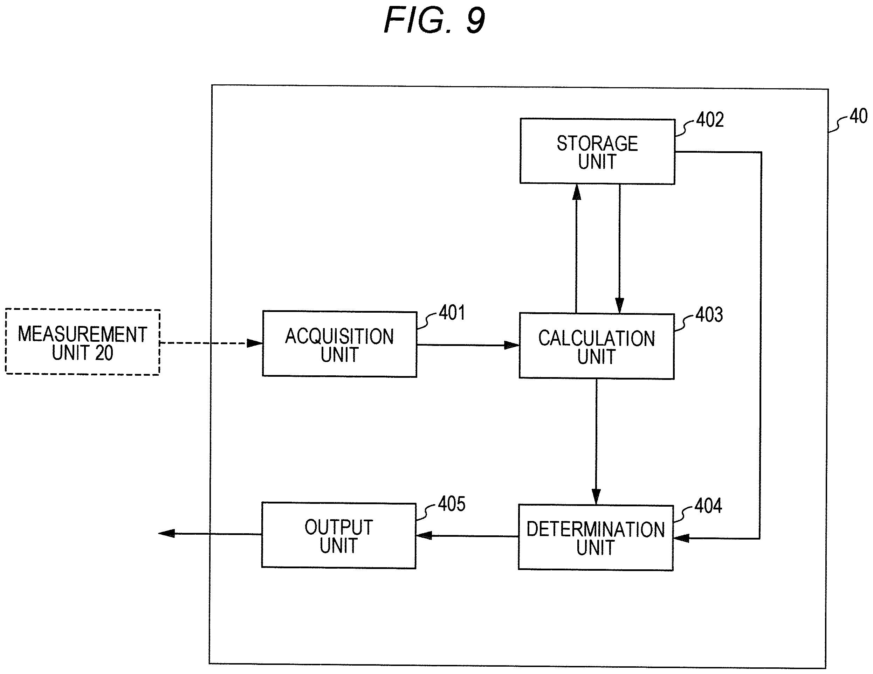

The measurement sample preparing unit includes the reagent tables 11 and 12, the cuvette table 13, the barcode reader 14, the cuvette supply section 15, the catcher 16, the specimen dispensing arm 17, the reagent dispensing arm 18, the urgent specimen setting section 19, the cuvette transfer section 23, the warming section 24, the waste port 25, the fluid section 26, and the specimen transporting section 30. Each of the reagent tables 11 and 12 and the cuvette table 13 has an annular shape. Each of the reagent tables 11 and 12 and the cuvette table 13 is configured rotatably. Each of the reagent tables 11 and 12 corresponds to a reagent storing section, onto which a reagent container 103 is placed. The barcode of the reagent container 103 placed on the reagent tables 11 and 12 is read by the barcode reader 14. Information (kind of reagent, reagent ID) read from the barcode is input to the control device 40 and stored in a hard disk 434 (see Fig. 9).

In the blood specimen analyzer of the present embodiment, a reagent container 103, in which a coagulation time measuring reagent, a calcium solution or the like is each accommodated, is placed on the reagent tables 11 and/or 12. Also, a reagent container 103, in which normal plasma is accommodated as a control specimen, may be placed on the reagent tables 11 and/or 12.

The cuvette table 13 is formed with a support portion 13a composed of a plurality of holes capable of supporting a cuvette 104. A new cuvette 104 introduced into the cuvette supply section 15 by the user is sequentially transferred by the cuvette supply section 15, and the cuvette 104 is placed on the support portion 13a of the cuvette table 13 by the catcher 16.

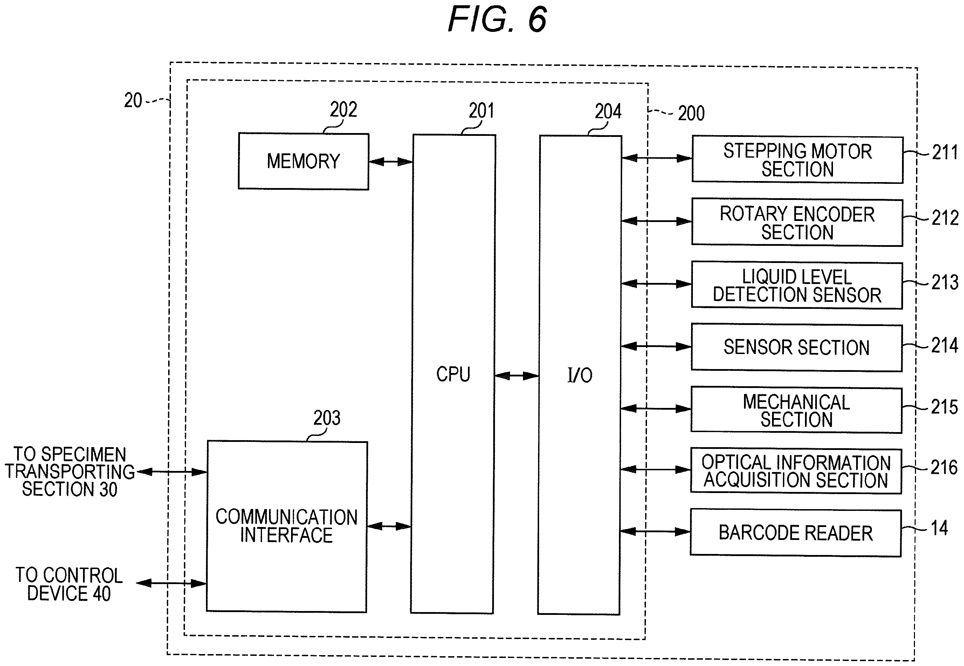

A stepping motor is connected to each of the specimen dispensing arm 17 and the reagent dispensing arm 18 so as to be able to move up and down and rotatably. A piercer 17a of which a tip is sharply formed is provided at the tip of the sample dispensing arm 17, so that the lid 101a of the specimen container 101 can be punctured. A pipette 18a is provided at the tip of the reagent dispensing arm 18. The tip of the pipette 18a is formed flat unlike the piercer 17a. An electrostatic capacitance type liquid level detection sensor 213 (see Fig. 6) is connected to the pipette 18a.

When the specimen container 101 is transported to a predetermined position by the specimen transporting unit 30 (see Fig. 4), the piercer 17a is positioned just above the specimen container 101 by the rotational movement of the specimen dispensing arm 17. Then, the specimen dispensing arm 17 is moved downward, the piercer 17a penetrates the lid 101a of the specimen container 101, and the blood specimen accommodated in the specimen container 101 is aspirated by the piercer 17a. When an urgent blood specimen is set in the urgent specimen setting section 19, the piercer 17a intervenes in the specimen supplied from the specimen transporting section 3 and aspirates the urgent blood specimen. The blood specimen aspirated by the piercer 17a is discharged into an empty cuvette 104 on the cuvette table 13.

The cuvette 104 into which the blood specimen has been discharged is transferred from the support portion 13a of the cuvette table 13 to a support portion 24a of the warming section 24 by a catcher 23a of the cuvette transfer section 23. The warming section 24 warms the blood specimen accommodated in the cuvette 104 placed in the support portion 24a at a predetermined temperature (for example, 36 to 38°C) for a certain period of time. When the warming of the blood specimen by the warming section 24 is finished, the cuvette 104 is again gripped by the catcher 23a. Then, the cuvette 104 is positioned at a predetermined position while being gripped by the catcher 23a, and in this state, the reagent aspirated by the pipette 18a is discharged into the cuvette 104.

In the dispensing of the reagent by the pipette 18a, first, the reagent tables 11 and 12 are rotated, and the reagent container 103 that accommodates the reagent corresponding to the measurement item is transported to an aspiration position by the pipette 18a. Then, after the position of the pipette 18a in the vertical direction is positioned at the origin position, the pipette 18a is lowered until the lower end of the pipette 18a comes into contact with the liquid level of the reagent by the liquid level detection sensor 213. When the lower end of the pipette 18a comes into contact with the liquid level of the reagent, the pipette 18a is further lowered to an extent that a necessary amount of the reagent can be aspirated. Then, the lowering of the pipette 18a is stopped, and the reagent is aspirated by the pipette 18a. The reagent aspirated by the pipette 18a is discharged into the cuvette 104 gripped by the catcher 23a. Then, the blood specimen and the reagent in the cuvette 104 are agitated by the vibrating function of the catcher 23a. Thus, the measurement sample is prepared. Thereafter, the cuvette 104 that accommodates the measurement sample is transferred to a support portion 22a of the detecting section 22 by the catcher 23a.

(Information Acquisition Unit)

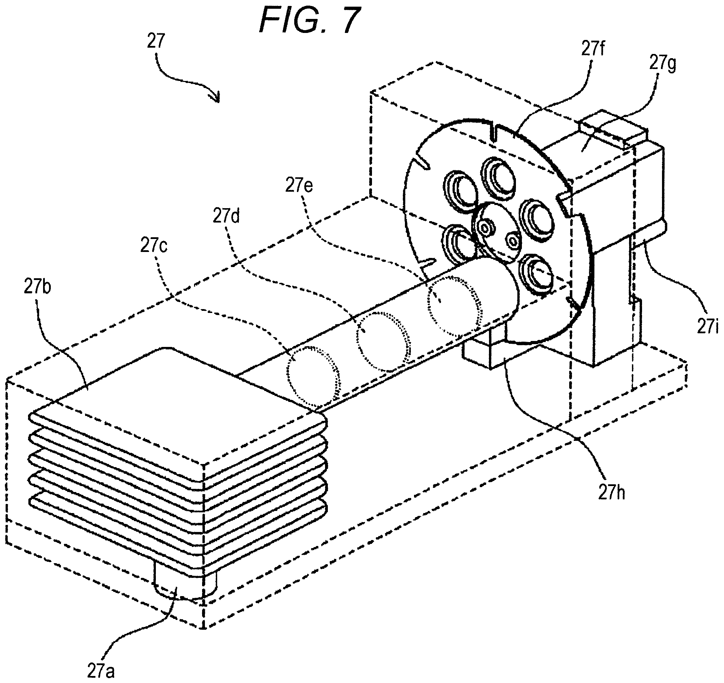

The information acquisition unit includes the optical fiber 21, the detecting section 22, and the lamp unit 27. The lamp unit 27 supplies light having plural kinds of wavelengths used for detection of an optical signal by the detecting section 22. An example of the configuration of the lamp unit 27 will be described with reference to Fig. 7. The lamp unit 27 corresponds to a light source. The lamp unit 27 includes a halogen lamp 27a, a lamp case 27b, condenser lenses 27c to 27e, a disk-shaped filter section 27f, a motor 27g, a light transmission type sensor 27h, and an optical fiber coupler 27i.

With reference to Fig. 5, light from the lamp unit 27 is supplied to the detecting section 22 via the optical fiber 21. A plurality of hole-shaped support portions 22a are provided in the detecting section 22, and a cuvette 104 can be inserted into each of the support portions 22a. The end part of the optical fiber 21 is attached to each of the support portions 22a, and the cuvette 104 supported by the support portion 22a can be irradiated with light from the optical fiber 21. The detecting section 22 irradiates the cuvette 104 with light supplied from the lamp unit 27 via the optical fiber 21. The detecting section 22 detects the light amount of light to be transmitted through the cuvette 104 (or scattered light from the cuvette 104).

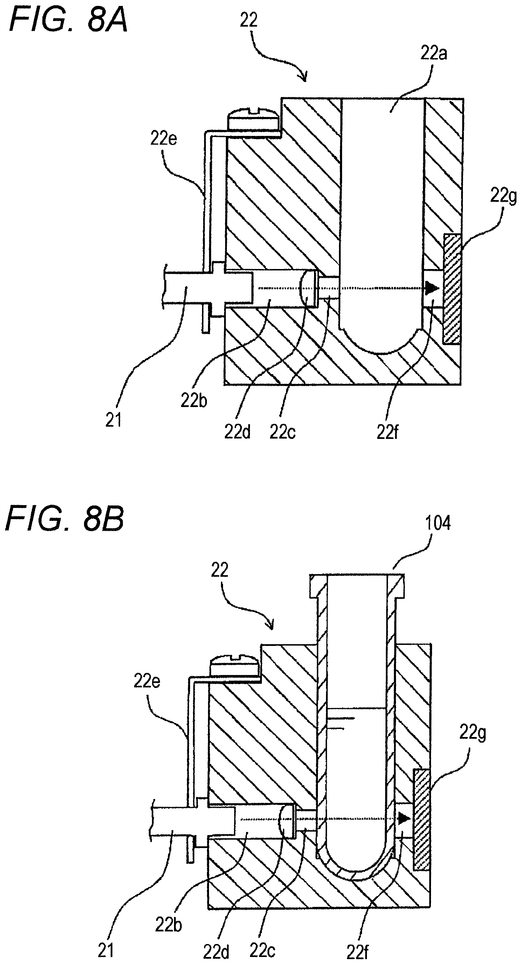

Figs. 8A to 8D show an example of one configuration of the plurality of support portions 22a arranged in the detecting section 22, and the other support portions 22a have the same configuration. With reference to Fig. 8A, the detecting section 22 is formed with a circular hole 22b into which the tip of the optical fiber 21 is inserted. The detecting section 22 is further formed with a circular communication hole 22c for communicating the hole 22b with the support portion 22a. The diameter of the hole 22b is larger than the diameter of the communication hole 22c. A lens 22d for condensing light from the optical fiber 21 is arranged at the end of the hole 22b. On the inner wall surface of the support portion 22a, a hole 22f is formed at a position facing the communication hole 22c. A photodetector 22g is arranged at the back of the hole 22f. The photodetector 22g corresponds to a light receiving portion. The photodetector 22g outputs an electric signal corresponding to the amount of received light. The light transmitted through the lens 22d is condensed on the light receiving surface of the photodetector 22g, through the communication hole 22c, the support portion 22a, and the hole 22f. The optical fiber 21 is prevented from falling off by a plate spring 22e in a state in which the end part of the optical fiber 21 is inserted into the hole 22b.

With reference to Fig. 8B, when the cuvette 104 is supported by the support portion 22a, the light condensed by the lens 22d is transmitted through the cuvette 104 and the sample accommodated in the cuvette 104, and the transmitted light enters the photodetector 22g. As the blood coagulation reaction progresses in the sample, the turbidity of the sample increases. Along with this, the amount of light to be transmitted through the sample (the amount of transmitted light) decreases, and the level of the detection signal of the photodetector 22g decreases.

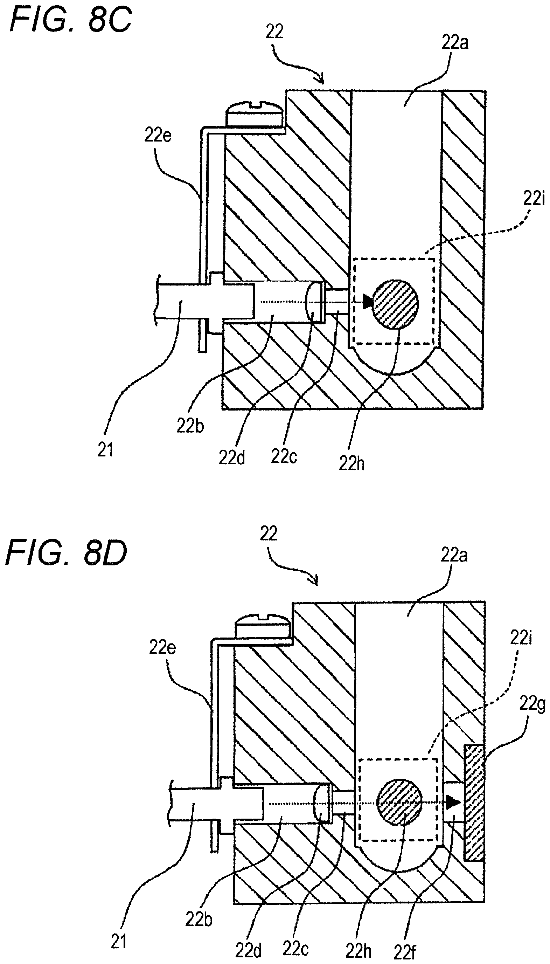

With reference to Fig. 8C, the configuration of the detecting section 22 when scattered light is used will be described. On the inner side surface of the support portion 22a, a hole 22h is provided at a position which is the same height as the communication hole 22c. A photodetector 22i is arranged at the back of the hole 22h. When the cuvette 104 is inserted into the support portion 22a and light is emitted from the optical fiber 21, the light scattered by the measurement sample in the cuvette 104 is irradiated to the photodetector 22i via the hole 22h. In this example, the detection signal from the photodetector 22i indicates the intensity of scattered light by the measurement sample. As shown in Fig. 8D, both the light to be transmitted through the measurement sample and the light to be scattered by the measurement sample may be detected.

As described above, the detecting section 22 irradiates the cuvette 104 with light supplied from the lamp unit 27. The detecting section 22 acquires optical information from the measurement sample. The acquired optical information is transmitted to the control device 40. The control device 40 performs analysis based on the optical information. The control device 40 displays the analysis result on a display unit 41.

After completion of the measurement, the cuvette 104 that has become unnecessary is transported by the cuvette table 13. The transported cuvette 104 is discarded to the disposal port 25 by the catcher 16. During the measurement operation, the piercer 17a and the pipette 18a are appropriately washed with a liquid such as a cleaning liquid supplied from the fluid section 26.

The hardware configuration of the measurement device will be described below. As shown in Fig. 6, the measurement unit 20 includes a control section 200, a stepping motor section 211, a rotary encoder section 212, a liquid level detection sensor 213, a sensor section 214, a mechanism section 215, an optical information acquisition section 216, and a barcode reader 14.

With reference to Fig. 6, the control section 200 includes a CPU 201, a memory 202, a communication interface 203, and an I/O interface 204. The CPU 201 executes a computer program stored in the memory 202. The memory 202 is composed of a ROM, a RAM, a hard disk, and the like. The CPU 201 drives the specimen transporting section 30 via the communication interface 203. The CPU 201 also transmits and receives instruction signals and data with the control device 40. The CPU 201 controls each section in the measurement unit 20 via the I/O interface 204. The CPU 201 also receives signals output from each section.

The stepping motor section 211 includes stepping motors for driving the reagent tables 11 and 12, the cuvette table 13, the catcher 16, the specimen dispensing arm 17, the reagent dispensing arm 18, and the cuvette transfer section 23, respectively. The rotary encoder section 212 includes a rotary encoder that outputs a pulse signal corresponding to the amount of rotational displacement of each stepping motor included in the stepping motor unit 211.

The liquid level detection sensor 213 is connected to the pipette 18a provided at the tip of the reagent dispensing arm 18. The liquid level detection sensor 213 detects that the lower end of the pipette 18a has come into contact with the liquid level of the reagent. The sensor section 214 includes a sensor for detecting that the vertical position of the pipette 18a is positioned at the origin position and a sensor for detecting that the power button 20d is pressed. The mechanism section 215 includes a mechanism for driving the cuvette supply section 15, the urgent specimen setting section 19, the warming section 24 and the fluid section 26, and an air pressure source which supplies pressure to the piercer 17a and the pipette 18a so that dispensing operation by the piercer 17a and the pipette 18a can be performed. With reference to Fig. 5, the optical information acquisition section 216 includes at least the lamp unit 27, the optical fiber 21, and the detecting section 22.

(Control Unit)

The configuration of the control device 40 (control unit) will be described below. As shown in Fig. 4, the control device 40 includes the display unit 41, an input unit 42, and a computer body 43. When the user inputs a measurement start instruction of a blood specimen via the input unit 42, the control device 40 transmits the measurement start instruction to the measurement unit 20 to start the measurement. The control device 40 receives optical information from the measurement unit 20. Then, a processor of the control device 40 calculates, based on the optical information, a primary differential curve of a coagulation waveform, parameters related to differentiation, and the like. The processor of the control device 40 may also calculate coagulation time based on the optical information. Then, the processor of the control device 40 executes a computer program for analyzing the blood specimen. Accordingly, the control device 40 also functions as a computer system for analyzing the blood specimen.

As to the functional configuration of the control device 40, as shown in Fig. 9, the control device 40 includes an acquisition unit 401, a storage unit 402, a calculation unit 403, a determination unit 404, and an output unit 405. The acquisition unit 401 is communicably connected to the measurement unit 20 via a network. The output unit 405 is communicably connected to the display unit 41.

The acquisition unit 401 acquires the optical information transmitted from the measurement unit 20. The storage unit 402 stores an expression for calculating values of various parameters related to differentiation of the coagulation waveform, a normal range corresponding to the various parameters, a predetermined reference value, and the like. The storage unit 402 may store an expression for calculating the coagulation time. Using the information acquired by the acquisition unit 401, the calculation unit 403 calculates the values of the various parameters according to the expression stored in the storage unit 402. The determination unit 404 determines whether or not the values of the parameters calculated by the calculation unit 403 deviates from the normal range corresponding to the parameters stored in the storage unit 402. The output unit 405 outputs the values of the parameters calculated by the calculation unit 403 as reference information regarding the blood specimen.

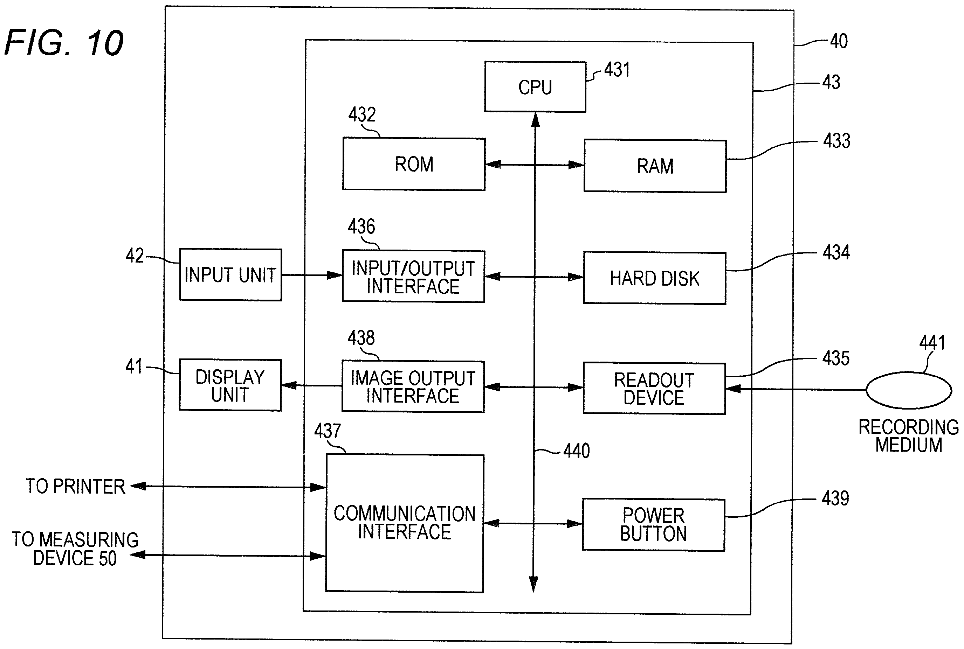

As shown in Fig. 10, the computer body 43 of the control device 40 includes a CPU 431, a ROM 432, a RAM 433, a hard disk 434, a readout device 435, an input/output interface 436, a communication interface 437, an image output interface 438, and a power button 439. The CPU 431, the ROM 432, the RAM 433, the hard disk 434, the readout device 435, the input/output interface 436, the communication interface 437, the image output interface 438, and the power button 439 are communicably connected by a bus 440.

The CPU 431 executes a computer program stored in the ROM 432 and a computer program loaded in the RAM 433. Each of the above-described functional blocks is realized by the CPU 431 executing an application program. Thus, the computer system functions as a terminal of the blood specimen analyzer.

The ROM 432 includes a mask ROM, PROM, EPROM, EEPROM, and the like. In the ROM 432, a computer program executed by the CPU 431 and data used for the computer program are recorded.

The RAM 433 includes SRAM, DRAM, and the like. The RAM 433 is used for reading out the computer program recorded in the ROM 432 and the hard disk 434. The RAM 433 is also used as a work area of the CPU 431 when executing these computer programs.

The hard disk 434 has installed therein an operating system, a computer program such as an application program (a computer program for analyzing a blood specimen) to be executed by the CPU 431, data used for executing the computer program, and setting contents of the control device 40.

The readout device 435 includes a flexible disk drive, a CD-ROM drive, a DVD ROM drive, and the like. The readout device 435 can read out a computer program or data recorded on a portable recording medium 441 such as a CD or a DVD.

The input/output interface 436 includes, for example, a serial interface such as USB, IEEE 1394 or RS-232C, a parallel interface such as SCSI, IDE or IEEE 1284, and an analog interface including a D/A converter, an A/D converter and the like. The input unit 42 such as a keyboard and a mouse is connected to the input/output interface 436. The user inputs an instruction via the input unit 42, and the input/output interface 436 receives a signal input via the input unit 42.

The communication interface 437 is, for example, an Ethernet (registered trademark) interface or the like. The control device 40 can transmit print data to a printer through the communication interface 437. The communication interface 437 is connected to the measurement unit 20, and the CPU 431 transmits and receives an instruction signal and data with the measurement unit 20 via the communication interface 437.

The image output interface 438 is connected to the display unit 41 including an LCD, a CRT, and the like. The image output interface 438 outputs a video signal corresponding to image data to the display unit 41, and the display unit 41 displays an image based on the video signal output from the image output interface 438.

With reference to Fig. 6, during the measurement operation, the CPU 201 of the measurement unit 20 temporarily stores in the memory 202 the data (optical information) obtained by digitizing the detection signal output from the detecting section 22 (see Fig. 5). The storage area of the memory 202 is divided into areas for each support portion 22a. In each area, the data (optical information) are sequentially stored which are acquired when the cuvette 104 supported by the corresponding support portion 22a is irradiated with light having a predetermined wavelength. Thus, the data is sequentially stored in the memory 202 over a predetermined measurement time. When the measurement time elapses, the CPU 201 stops storing the data in the memory 202, and the CPU 201 transmits the stored data to the control device 40 via the communication interface 203. The control device 40 processes and analyzes the received data. The control device 40 displays the analysis result on the display unit 41.

(Processing Procedure of Blood Specimen Analyzer)

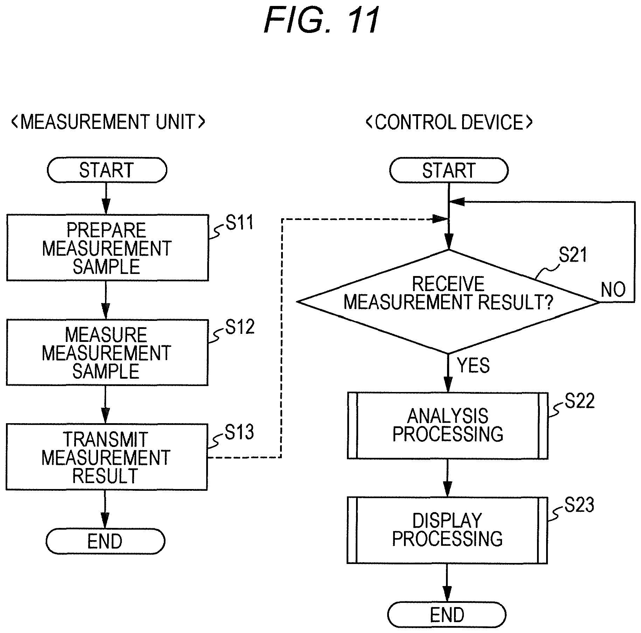

The processing in the measurement unit 20 is mainly performed under the control of the CPU 201 of the measurement unit 20, and the processing in the control device 40 is mainly performed under the control of the CPU 431 of the control device 40. When receiving a measurement start instruction input by the user from the control device 40, the measurement unit 20 starts measurement processing. With reference to Fig. 11, when the measurement processing is started, the measurement unit 20 aspirates a predetermined amount of a blood specimen from the specimen container 101 transported by the specimen transporting section, and the measurement unit 20 dispenses the aspirated blood specimen into an empty cuvette 104 on the cuvette table 13. When also measuring normal plasma as a control sample, the measurement unit 20 aspirates a predetermined amount of the normal plasma from a reagent container 103 containing the normal plasma accommodated in the reagent accommodation section, and the measurement unit 20 dispenses the aspirated blood specimen into an empty cuvette 104. The measurement unit 20 transfers the cuvette 104 into which the specimen is dispensed to the warming section 24. The measurement unit 20 warms the plasma in the cuvette 104 to a predetermined temperature (for example, 37°C). Thereafter, the measurement unit 20 adds a coagulation time measuring reagent and a calcium solution to the cuvette 104 to prepare a measurement sample (step S11).

The measurement unit 20 transfers the cuvette 104 to which various reagents are added to the detecting section 22. The measurement unit 20 irradiates the cuvette 104 with light to measure the measurement sample (step S12). The measurement unit 20 starts measuring the time from the start point, preferably the time from the time point when the calcium solution is added to the cuvette 104. In this measurement, data (the amount of scattered light or the amount of transmitted light) based on the light with a wavelength of 660 nm is sequentially stored in the memory 202 during the measurement time. At this time, the data is stored in the memory 202 in a state associated with the elapsed time from the start point, preferably the elapsed time from the time point of adding the calcium solution. Then, when the measurement time elapses, the measurement unit 20 stops the measurement, and the measurement unit 20 transmits the measurement result (data) stored in the memory 202 to the control device 40 (step S13). Accordingly, when the control device 40 receives the measurement result (data) from the measurement unit 20 (step S21: YES), the control device 40 executes analysis processing on the received measurement result (step S22). That is, the control device 40 calculates, regarding the measurement sample, parameters related to differentiation of a coagulation waveform from the coagulation waveform. The control device 40 may calculate the coagulation time of the measurement sample, the coagulation waveform, the waveform of rate, and the like. After performing the analysis processing, the control device 40 executes display processing of the analysis result (step S23).

(Processing Procedure for Acquiring Average Change Rate of Coagulation Rate and Output of Information on Severity of Hemophilia)

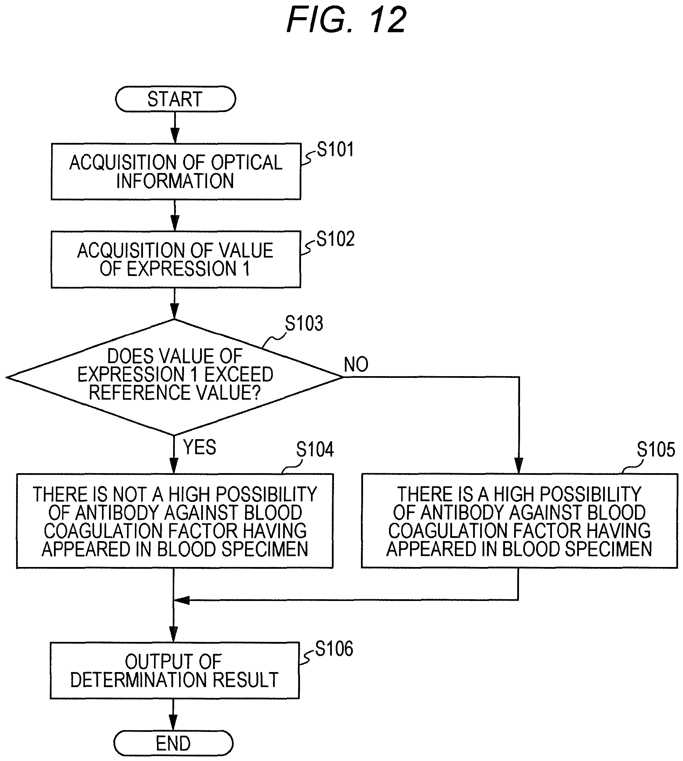

With reference to Fig. 12, an example of the processing flow for acquiring the average change rate of the coagulation rate will be described. However, the present embodiment is not limited to this example.

In step S101, the acquisition unit 401 of the control device 40 acquires optical information (scattered light intensity, transmittance or absorbance) based on the data (the amount of scattered light or the amount of transmitted light) received from the measurement unit 20. In step S102, the calculation unit 403 acquires a coagulation waveform from the optical information acquired by the acquisition unit 401. The calculation unit 403 calculates parameters related to differentiation of the coagulation waveform according to the expression stored in the storage unit 402. The calculation unit 403 may further calculate the coagulation time, the coagulation waveform, the waveform of rate and the like from the optical information acquired by the acquisition unit 401. Then, the calculation unit 403 calculates, based on the calculation results and the like, the value of Expression 1 according to Expression 1 above stored in the storage unit 402.

In step S103, the determination unit 404 determines whether or not the value of Expression 1 calculated by the calculation unit 403 exceeds the reference value stored in the storage unit 402. When the value of Expression 1 exceeds the reference value, the processing proceeds to step S104. In step S104, the determination unit 404 transmits to the output unit 405 a determination result that there is not a high possibility of the antibody against a coagulation factor having appeared in the blood specimen. On the other hand, when the value of Expression 1 is equal to or less than the reference value, the processing proceeds to step S105. In step S105, the determination unit 404 transmits to the output unit 405 a determination result that there is a high possibility of the antibody against a coagulation factor having appeared in the blood specimen.

In step S106, the output unit 405 outputs the determination result, the output unit 405 displays the determination result on the display unit 41, or the output unit 405 makes a printer to print the determination result. Alternatively, the determination result may be output by voice. Thus, the determination result can be provided to the user as reference information on the blood specimen.

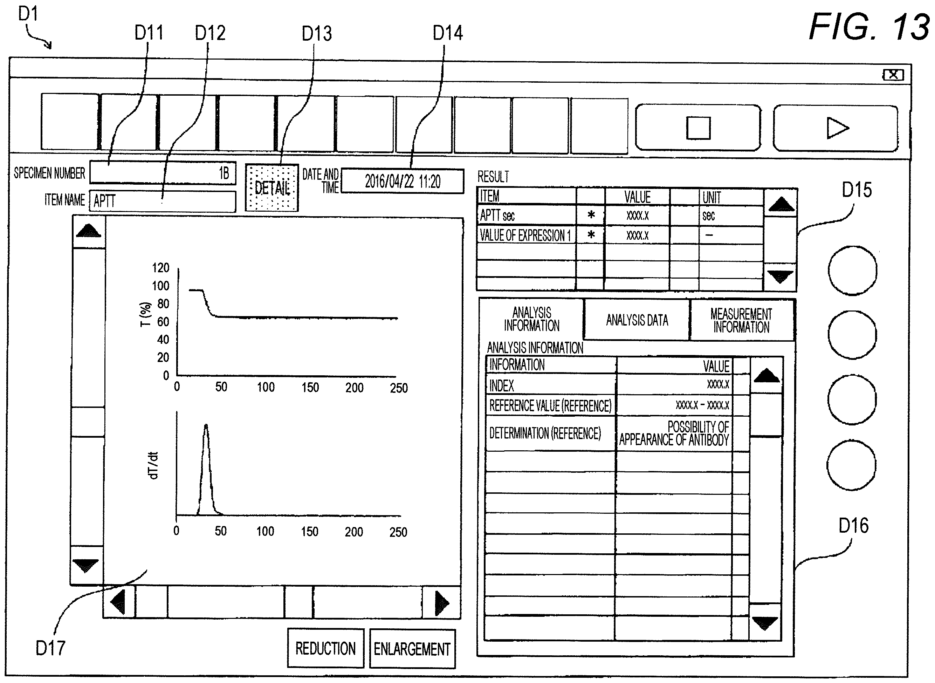

As an example of a screen displaying the analysis result, a screen for displaying the result of analyzing the process of coagulation of a blood specimen using an APTT measuring reagent and a calcium solution will be described with reference to Fig. 13. A screen D1 includes an area D11 for displaying a specimen number, an area D12 for displaying a measurement item name, a button D13 for displaying a detailed screen, an area D14 for displaying measurement data and time, an area D15 for displaying measurement results, an area D16 for displaying analysis information, and an area D17 for displaying a coagulation waveform and its primary differential curve.

In the area D15, measurement items and measurement values are displayed. In the area D15, "APTT sec" is the activated partial thromboplastin time. In the area D15, the values of the parameters related to differentiation of the coagulation waveform such as the value of Expression 1 and |min1| may be displayed.

In the area D16, analysis items and reference information are displayed. In the area D16, "Index" is the value of the parameter related to differentiation of the coagulation waveform used for the determination. "Reference Value (Reference)" is a reference value corresponding to the parameter value used for the determination. "Determination (Reference)" is a determination result by the blood specimen analyzer. In Fig. 13, it is shown that there is a high possibility of the antibody against a coagulation factor having appeared in the blood specimen. It is desirable that the diagnosis as to whether or not the antibody against a coagulation factor has appeared in a patient is performed with consideration of not only this judgment result but also information such as other inspection results. Accordingly, "(Reference)" is displayed to indicate that the determination result by the blood specimen analyzer according to the present embodiment and the reference value are reference information. In Fig. 13, the determination result is displayed by the letters "Possibility of Appearance of Antibody", but the determination result may be displayed with a symbol such as a flag or a graphic indicator. Alternatively, the determination result may be output by voice.

Hereinafter, the present invention will be described with reference to examples, but is not limited to these examples.

EXAMPLES

Example: Discrimination by Average Change Rate of Coagulation Rate

(1) Reagent

As commercially available APTT measuring reagents, Thrombocheck APTT-SLA (Sysmex Corporation) and Actin FS (Siemens Healthcare Diagnostics Inc.) were used. As a coagulation initiation reagent containing calcium ions, a 20 mM calcium chloride solution (Sysmex Corporation) was used.

(2) Blood specimen . Severe hemophilia A plasma specimen 10 specimens (Severe Haemophilia A; factor VIII activity < 1.0 IU/dl):

VS-HA specimen 5 specimens (factor VIII activity < 0.2 IU/dl)
MS-HA specimen 5 specimens (factor VIII activity 0.2 IU/dl to 1.0 IU/dl) . Hemophilia A specimen in which the antibody has appeared 10 specimens:
HA-inh 10 specimens (factor VIII activity < 0.2 IU/dl)

(3) Preparation and measurement of measurement sample

For preparing and measuring a measurement sample, a fully automated blood coagulation measurement device CS-2000i (Sysmex Corporation) was used. A blood coagulation analyzing reagent (50 $\mu$L) was added to a plasma specimen (50 $\mu$L), and the mixture was incubated at 37°C for 3 minutes. Then, a measurement sample was prepared by adding a 20 mM calcium chloride solution (50 $\mu$L). The transmittance of the measurement sample was continuously measured for 420 seconds from the addition of the calcium chloride solution. The following plasma specimens were used.

(4) Analysis results

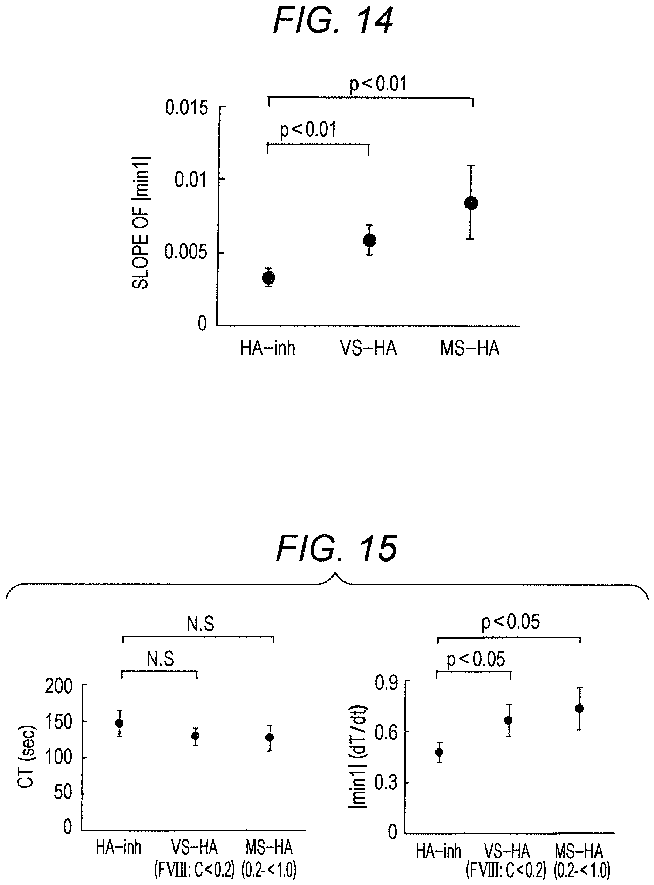

A change with time in transmittance was plotted to obtain a coagulation waveform. The coagulation waveform data was primarily differentiated to obtain waveform data of rate. In addition, time to |min1|, |min1| and time to |min2| were calculated as the coagulation time and the parameters related to differentiation of the coagulation waveform. These parameters were applied to the following Expression 1 to calculate the average change rate of the coagulation rate. The results are shown in Fig. 14.
[Mathematical Expression 2]

$$(1/2 \times |\text{min1}|)/(\text{time to } |\text{min1}| - \text{time to } |\text{min2}|)...\text{Expression 1}$$

As is apparent from Fig. 14, the specimen (HA-inh) in which the antibody has appeared and the severe hemophilia A specimens (VS-HA, MS-HA) could be distinguished with very high accuracy ($p < 0.01$) by comparing each average change rate of the coagulation rate calculated with the Expression 1 above. As shown in a comparative example described later, these specimens are distinguished with $p < 0.05$ with known parameters. The above results are superior in that it is possible to distinguish specimens (HA-inh) in which the antibody has appeared and the severe hemophilia A specimens (VS-HA, MS-HA) with higher accuracy than known parameters. According to the discrimination by the parameters calculated by Expression 1 above, the probabilities of false positives and false negatives can be suppressed. As a result, VS-HA with a factor VIII activity of less than 0.2 IU/dl and hemophilia in which the antibody has appeared can be discriminated with higher accuracy than before, and thus it is possible to select a treatment policy suitable for each patient.

Comparative Example: Discrimination by Maximum Coagulation Rate |min1|

With respect to the specimen (HA-inh) in which the antibody has appeared and the severe hemophilia A specimens (VS-HA, MS-HA), the parameters related to differentiation acquired in the coagulation waveform analysis in the above example |min1| (maximum coagulation rate) were compared. The results are shown in Fig. 15.
As is apparent from Fig. 15, the specimen (HA-inh) in which the antibody has appeared and the severe hemophilia A specimens (VS-HA, MS-HA) could be distinguished ($p < 0.05$) by comparing each of the maximum coagulation rates. However, the specimens could not be distinguished with p of less than 0.01. That is, the discrimination based on the maximum coagulation rate is not so highly accurate as the discrimination results based on the parameter calculated by Expression 1. In the discrimination based on the maximum coagulation rate, the probabilities of false positives and false negatives cannot be suppressed as the discrimination is made by the parameter calculated with Expression 1.

**Claims**

1. A method for determining severity of hemophilia, the method comprising the steps of:

coagulating a blood specimen to acquire a coagulation waveform;

acquiring an average change rate of a coagulation rate from the coagulation waveform, wherein the average change rate of the coagulation rate is calculated based on a waveform of rate obtained by differentiating the coagulation waveform; and
determining the severity of hemophilia in the blood specimen based on the average change rate of the coagulation rate.

2. The method for determining severity of hemophilia according to claim 1, wherein the average change rate of the coagulation rate is a magnitude of a slope of a straight line connecting two points on the waveform of rate.

3. The method for determining severity of hemophilia according to claim 1 or 2, wherein the average change rate of the coagulation rate is calculated using a maximum coagulation rate (|min1|) and a time until the coagulation rate becomes a maximum (time to |min1|).

4. The method for determining severity of hemophilia according to claim 3, wherein the average change rate of the coagulation rate is calculated by further using a time until coagulation acceleration becomes a maximum (time to |min2|).

5. The method for determining severity of hemophilia according to claim 4, wherein the average change rate of the coagulation rate is calculated by Expression 1 below:

$$(1/2 \times |min1|)/(time\ to\ |min1| - time\ to\ |min2|)...Expression\ 1.$$

6. The method for determining severity of hemophilia according to claim 4 or 5, wherein the time until the coagulation acceleration becomes the maximum is a time before the coagulation rate becomes the maximum and until the coagulation rate becomes 1/2 of the maximum coagulation rate.

7. The method for determining severity of hemophilia according to any one of claims 1 to 6, wherein when the average change rate of the coagulation rate is equal to or less than a reference value, it is determined that there is a high possibility of antibody against a coagulation factor having appeared in the blood specimen.

8. The method for determining severity of hemophilia according to any one of claims 1 to 7, wherein the blood specimen is a blood specimen derived from a patient suspected of being a Very-Severe Haemophilia A (VS-HA) patient with less than 0.2% FVIII activity, and
when the average change rate of the coagulation rate is equal to or less than the reference value, it is determined that there is a high possibility of antibody against the coagulation factor having appeared in the blood specimen, and when the average change rate of the coagulation rate exceeds the reference value, it is determined that there is not a high possibility of the blood specimen being a blood specimen of a VS-HA patient.

9. The method for determining severity of hemophilia according to any one of claims 1 to 8, wherein the severity of hemophilia is severity of hemophilia A.

10. The method for determining severity of hemophilia according to any one of claims 1 to 9, wherein the coagulation waveform is acquired based on optical information obtained by preparing a measurement sample by contacting the blood specimen with a coagulation time measuring reagent, and irradiating the measurement sample with light.

11. The method for determining severity of hemophilia according to any one of claims 1 to 10, wherein the coagulation time measuring reagent is a reagent for measuring activated partial thromboplastin time.

12. The method for determining severity of hemophilia according to any one of claims 1 to 11, wherein the optical information is light transmittance, an amount of scattered light, or absorbance.

13. A blood specimen analyzer comprising:

a measurement sample preparing unit configured to prepare a measurement sample including a blood specimen and a coagulation time measuring reagent;
an information acquisition unit configured to acquire a coagulation waveform from the prepared measurement sample; and
a control unit,
wherein
the control unit is configured to control the measurement sample preparing unit so as to prepare the measurement sample from the blood specimen and the coagulation time measuring reagent, is configured to acquire an average change rate of a coagulation rate based on the coagulation waveform, wherein the average change rate of the coagulation rate is calculated based on a waveform of rate obtained by differentiating the coagulation waveform, and is configured to output information on severity of hemophilia based on the average change rate of the coagulation rate.

**Patentansprüche**

1. Verfahren zum Bestimmen des Schweregrads von Hämophilie, wobei das Verfahren die Schritte umfasst:

   Koagulieren einer Blutprobe, um eine Koagulationswellenform zu erfassen;
   Erfassen einer durchschnittlichen Änderungsrate einer Koagulationsgeschwindigkeit aus der Koagulationswellenform, wobei die durchschnittliche Änderungsrate der Koagulationsgeschwindigkeit basierend auf einer Wellenform der Geschwindigkeit, erhalten durch Differenzieren der Koagulationswellenform, berechnet wird; und
   Bestimmen des Schweregrads von Hämophilie in der Blutprobe basierend auf der durchschnittlichen Änderungsrate der Koagulationsgeschwindigkeit.

2. Verfahren zum Bestimmen des Schweregrads von Hämophilie gemäß Anspruch 1, wobei die durchschnittliche Änderungsrate der Koagulationsgeschwindigkeit die Größe einer Steigung einer geraden Linie ist, welche zwei Punkte auf der Wellenform der Geschwindigkeit verbindet.

3. Verfahren zum Bestimmen des Schweregrads von Hämophilie gemäß Anspruch 1 oder 2, wobei die durchschnittliche Änderungsrate der Koagulationsgeschwindigkeit unter Verwendung einer maximalen Koagulationsgeschwindigkeit (|min1|) und einer Zeit, bis die Koagulationsgeschwindigkeit ein Maximum erreicht (Zeit bis |min1|), berechnet wird.

4. Verfahren zum Bestimmen des Schweregrads von Hämophilie gemäß Anspruch 3, wobei die durchschnittliche Änderungsrate der Koagulationsgeschwindigkeit weitergehend unter Verwendung einer Zeit, bis die Koagulationsbeschleunigung ein Maximum erreicht (Zeit bis |min2|), berechnet wird.

5. Verfahren zum Bestimmen des Schweregrads von Hämophilie gemäß Anspruch 4, wobei die durchschnittliche Änderungsrate der Koagulationsgeschwindigkeit durch Ausdruck 1 hierunter berechnet wird:

   ```
   (1/2 × |min1|)/(Zeit bis |min1| - Zeit bis
   |min2|)...Ausdruck 1.
   ```

6. Verfahren zum Bestimmen des Schweregrads von Hämophilie gemäß Anspruch 4 oder 5, wobei die Zeit, bis die Koagulationsbeschleunigung das Maximum erreicht, eine Zeit ist, bevor die Koagulationsgeschwindigkeit das Maximum erreicht, und bis die Koagulationsgeschwindigkeit 1/2 der maximalen Koagulationsgeschwindigkeit erreicht.

7. Verfahren zum Bestimmen des Schweregrads von Hämophilie gemäß irgendeinem der Ansprüche 1 bis 6, wobei, wenn die durchschnittliche Änderungsrate der Koagulationsgeschwindigkeit gleich einem oder kleiner als ein Referenzwert ist, bestimmt wird, dass eine hohe Wahrscheinlichkeit vorliegt, dass ein Antikörper gegen einen Koagulationsfaktor in der Blutprobe aufgetreten ist.

8. Verfahren zum Bestimmen des Schweregrads von Hämophilie gemäß irgendeinem der Ansprüche 1 bis 7, wobei die Blutprobe eine von einem Patienten, bei welchem der Verdacht besteht, ein sehr schwerer Hämophilie A (Very-severe haemophilia A, VS-HA)-Patient mit weniger als 0,2% FVIII-Aktivität zu sein, erhaltene Blutprobe ist, und, wenn die durchschnittliche Änderungsrate der Koagulationsgeschwindigkeit gleich dem oder weniger als der Referenzwert ist, bestimmt wird, dass eine hohe Wahrscheinlichkeit vorliegt, dass ein Antikörper gegen den Koagulationsfaktor in der Blutprobe aufgetreten ist, und, wenn die durchschnittliche Änderungsrate der Koagulationsgeschwindigkeit den Referenzwert übersteigt, bestimmt wird, dass keine hohe Wahrscheinlichkeit vorliegt, dass die Blutprobe eine Blutprobe eines VS-HA-Patienten ist.

9. Verfahren zum Bestimmen des Schweregrads von Hämophilie gemäß irgendeinem der Ansprüche 1 bis 8, wobei der Schweregrad von Hämophilie Schweregrad von Hämophilie A ist.

10. Verfahren zum Bestimmen des Schweregrads von Hämophilie gemäß irgendeinem der Ansprüche 1 bis 9, wobei die Koagulationswellenform basierend auf optischer Information, erhalten durch Vorbereiten einer Messprobe durch Inkontaktbringen der Blutprobe mit einem Koagulationszeit-Messreagens, und Bestrahlen der Messprobe mit Licht, erfasst wird.

11. Verfahren zum Bestimmen des Schweregrads von Hämophilie gemäß irgendeinem der Ansprüche 1 bis 10, wobei das Koagulationszeit-Messreagens ein Reagens zum Messen von aktivierter partieller Thromboplastinzeit ist.

12. Verfahren zum Bestimmen des Schweregrads von Hämophilie gemäß irgendeinem der Ansprüche 1 bis 11, wobei die optische Information Lichttransmission, eine Menge an Streulicht oder Absorbanz ist.

13. Blutprobenanalysegerät, umfassend:

eine Messproben-Vorbereitungseinheit, welche konfiguriert ist, um eine Messprobe, welche eine Blutprobe und ein Koagulationszeit-Messreagens enthält, vorzubereiten;
eine Informationserfassungseinheit, welche konfiguriert ist, um eine Koagulationswellenform aus der vorbereiteten Messprobe zu erfassen; und
eine Steuereinheit,
wobei
die Steuereinheit konfiguriert ist, um die Messproben-Vorbereitungseinheit so zu steuern, dass die Messprobe aus der Blutprobe und dem Koagulationszeit-Messreagens vorbereitet wird, konfiguriert ist, um basierend auf der Koagulationswellenform eine durchschnittliche Änderungsrate der Koagulationsgeschwindigkeit zu erfassen, wobei die durchschnittliche Änderungsrate der Koagulationsgeschwindigkeit basierend auf einer Wellenform der Geschwindigkeit, erhalten durch Differenzieren der Koagulationswellenform, berechnet wird, und konfiguriert ist, um Information über den Schweregrad von Hämophilie basierend auf der durchschnittlichen Änderungsrate der Koagulationsgeschwindigkeit auszugeben.

## Revendications

1. Méthode pour déterminer la sévérité de l'hémophilie, la méthode comprenant les étapes de :

coagulation d'un échantillon de sang pour acquérir une forme d'onde de coagulation ;
acquisition d'une vitesse de changement moyenne d'une vitesse de coagulation à partir de la forme d'onde de coagulation, dans laquelle la vitesse de changement moyenne est calculée sur la base d'une forme d'onde de vitesse obtenue en différenciant la forme d'onde de coagulation; et
détermination de la sévérité de l'hémophilie dans l'échantillon de sang sur la base de la vitesse de changement moyenne de la vitesse de coagulation.

2. La méthode pour déterminer la sévérité de l'hémophilie selon la revendication 1, dans laquelle la vitesse de changement moyenne est une magnitude d'une pente d'une ligne droite connectant deux points sur la forme d'onde de vitesse.

3. La méthode pour déterminer la sévérité de l'hémophilie selon la revendication 1 ou 2, dans laquelle la vitesse de changement moyenne de la vitesse de coagulation est calculée en utilisant une vitesse de coagulation maximale ($|min1|$) et un temps jusqu'à ce que la vitesse de coagulation atteigne un maximum (temps jusqu'à $|mini1|$).

4. La méthode pour déterminer la sévérité de l'hémophilie selon la revendication 3, dans laquelle la vitesse de changement moyenne de la vitesse de coagulation est calculée en utilisant en outre un temps jusqu'à ce qu'une accélération de coagulation atteigne un maximum (temps jusqu'à $|min2|$).

5. La méthode pour déterminer la sévérité de l'hémophilie selon la revendication 4, dans laquelle la vitesse de changement moyenne de la vitesse de coagulation est calculée par l'Expression 1 ci-dessous:

$$(1/2 \times |min1|)/(\text{temps jusqu'à } |min1| - \text{temps jusqu'à } |min2|)...\text{Expression 1.}$$

6. La méthode pour déterminer la sévérité de l'hémophilie selon la revendication 4 ou 5, dans laquelle le temps jusqu'à ce que l'accélération de coagulation atteigne le maximum est un temps avant que la vitesse de coagulation atteigne le maximum et jusqu'à ce que la vitesse de coagulation atteigne 1/2 de la vitesse de coagulation maximale.

7. La méthode pour déterminer la sévérité de l'hémophilie selon l'une quelconque des revendications 1 à 6, dans laquelle la vitesse de changement moyenne de la vitesse de coagulation est égale à ou inférieure à une valeur de référence, il est déterminé qu'il y a une haute possibilité d'anticorps contre un facteur de coagulation ayant apparu dans l'échantillon de sang.

8. La méthode pour déterminer la sévérité de l'hémophilie selon l'une quelconque des revendications 1 à 7, dans laquelle l'échantillon de sang est un échantillon de sang d'un patient suspecté d'être un patient à hémophilie A très sévère (VS-HA) avec une activité FVIII inférieure à 0.2%, et
lorsque la vitesse de changement moyenne de la vitesse de coagulation est égale à ou inférieure à la valeur de référence, il est déterminé qu'il y a une haute possibilité d'anticorps contre le facteur de coagulation ayant apparu dans l'échantillon de sang, et lorsque la vitesse de changement moyenne de la vitesse de coagulation excède la valeur de référence, il est déterminé qu'il n'y a pas une haute possibilité que l'échantillon de sang soit un échantillon de sang d'un patient VS-HA.

9. La méthode pour déterminer la sévérité de l'hémophilie selon l'une quelconque des revendications 1 à 8, dans laquelle la sévérité de l'hémophilie est la sévérité de l'hémophilie A.

10. La méthode pour déterminer la sévérité de l'hémophilie selon l'une quelconque des revendications 1 à 9, dans laquelle l'onde de forme de coagulation est acquise sur la base d'information optique obtenue en préparant un échantillon de mesure par mise en contact de l'échantillon de sang avec un réactif de mesure du temps de coagulation, et irradiation de l'échantillon de mesure avec de la lumière.

11. La méthode pour déterminer la sévérité de l'hémophilie selon l'une quelconque des revendications 1 à 10, dans laquelle le réactif de mesure du temps de coagulation est un réactif pour mesurer le temps de thromboplastine partielle activée.

12. La méthode pour déterminer la sévérité de l'hémophilie selon l'une quelconque des revendications 1 à 11, dans laquelle l'information optique est la transmittance lumineuse, une quantité de lumière dispersée, ou une absorbance.

13. Analyseur d'échantillon de sang comprenant :

une unité de préparation d'échantillon de mesure configure pour préparer un échantillon de mesure comportant un échantillon de sang et un réactif de mesure du temps de coagulation;
une unité d'acquisition d'information configure pour acquérir une onde de forme de coagulation à partir de l'échantillon de mesure préparé ; et
une unité de contrôle,
dans lequel
l'unité de contrôle est configurée pour contrôler l'unité de préparation d'échantillon de mesure de sorte à préparer l'échantillon de mesure à partir de l'échantillon de sang et du réactif de mesure du temps de coagulation, est configurée pour acquérir une vitesse de changement moyenne d'une vitesse de coagulation sur la base de la forme d'onde de coagulation, dans laquelle la vitesse de changement moyenne de la vitesse de coagulation est calculée sur la base d'une forme d'onde de vitesse obtenue en différenciant la forme d'onde de coagulation, et est configurée pour fournir une information sur la sévérité de l'hémophilie sur la base de la vitesse de changement moyenne de la vitesse de coagulation.

## FIG. 1A

## FIG. 1B

## FIG. 2

## FIG. 3

FIG. 4

FIG. 5

*FIG. 6*

EP 3 276 356 B1

## FIG. 7

27
27f
27g
27e
27d
27c
27b
27i
27h
27a

EP 3 276 356 B1

## FIG. 8A

## FIG. 8B

## FIG. 8C

22
22a
22e
22i
21
22b
22d
22c
22h

## FIG. 8D

22
22a
22e
22i
22g
21
22b
22d
22c
22h
22f

## FIG. 9

EP 3 276 356 B1

*FIG. 10*

CPU — 431

432 — ROM ⟷ RAM — 433

436 — INPUT/OUTPUT INTERFACE

42 — INPUT UNIT

HARD DISK — 434

438 — IMAGE OUTPUT INTERFACE

41 — DISPLAY UNIT

READOUT DEVICE — 435

RECORDING MEDIUM — 441

440

437 — COMMUNICATION INTERFACE

TO PRINTER

TO MEASURING DEVICE 50

POWER BUTTON — 439

40
43

27

EP 3 276 356 B1

# FIG. 11

〈MEASUREMENT UNIT〉

〈CONTROL DEVICE〉

```
        START
          │
   ┌──────────────┐
   │   PREPARE    │  S11
   │ MEASUREMENT  │
   │    SAMPLE    │
   └──────────────┘
          │
   ┌──────────────┐
   │   MEASURE    │  S12
   │ MEASUREMENT  │
   │    SAMPLE    │
   └──────────────┘
          │
   ┌──────────────┐
   │  TRANSMIT    │  S13
   │ MEASUREMENT  │
   │   RESULT     │
   └──────────────┘
          │
        END
```

```
          START
            │
          ◇ S21
   RECEIVE
MEASUREMENT RESULT?  ── NO
            │ YES
   ┌──────────────┐
   │  ANALYSIS    │  S22
   │ PROCESSING   │
   └──────────────┘
            │
   ┌──────────────┐
   │   DISPLAY    │  S23
   │ PROCESSING   │
   └──────────────┘
            │
          END
```

# FIG. 12

```
                    ┌──────────────┐
                    │    START     │
                    └──────┬───────┘
                           │
                           ▼
              ┌──────────────────────────┐  S101
              │  ACQUISITION OF OPTICAL   │
              │       INFORMATION         │
              └────────────┬─────────────┘
                           │
                           ▼
              ┌──────────────────────────┐  S102
              │   ACQUISITION OF VALUE    │
              │      OF EXPRESSION 1      │
              └────────────┬─────────────┘
                           │
                           ▼
                    S103
              ╱─────────────────────╲
             ╱   DOES VALUE OF        ╲   NO
            ╱  EXPRESSION 1 EXCEED      ╲──────────────┐
            ╲   REFERENCE VALUE?        ╱              │
             ╲                         ╱               │
              ╲───────────┬───────────╱                │
                          │ YES                        │
                  S104    │                    S105    │
                          ▼                            ▼
    ┌──────────────────────────────────┐  ┌──────────────────────────────────┐
    │   THERE IS NOT A HIGH POSSIBILITY │  │     THERE IS A HIGH POSSIBILITY   │
    │   OF ANTIBODY AGAINST BLOOD       │  │    OF ANTIBODY AGAINST BLOOD      │
    │   COAGULATION FACTOR HAVING       │  │    COAGULATION FACTOR HAVING      │
    │   APPEARED IN BLOOD SPECIMEN      │  │    APPEARED IN BLOOD SPECIMEN     │
    └───────────────┬──────────────────┘  └──────────────┬───────────────────┘
                    │                                     │
                    │◄────────────────────────────────────┘
                    │
                    ▼
              ┌──────────────────────────┐  S106
              │       OUTPUT OF          │
              │  DETERMINATION RESULT    │
              └────────────┬─────────────┘
                           │
                           ▼
                    ┌──────────────┐
                    │     END      │
                    └──────────────┘
```

FIG. 13

## FIG. 14

## FIG. 15

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2013344519 A1
- WO 2011159820 A1
- US 2016178651 A1

### Non-patent literature cited in the description

- **MATSUMOTO et al.** A putative inhibitory mechanism in the tenase complex responsible for loss of coagulation function in acquired hemophilia A patients with anti-C2 autoantibodies. *Thorombosis and Haemostasis,* February 2012, 107
- **MATSUMOTO et al.** *J Thromb Haemost,* 2006, vol. 4 (2), 377-84
- **CHITLUR.** *Thromb Res,* 2012, vol. 130 (1), 1-6
- **YADA et al.** *Thromb Haemost,* 2013, vol. 109 (6), 1007-15